# EUROPEAN PATENT SPECIFICATION

(11) **EP 0 498 737 B1**
(45) Date of publication and mention of the grant of the patent: **19.07.1995**
(21) Application number: 92400329.6
(22) Date of filing: 07.02.1992
(51) Int. Cl.: A61M 5/315

(54) **Dosing means for an injector**
Dosiereinrichtung für Injektor
Dispositif de dosage pour injecteur

(30) Priority: 07.02.1991 JP 16326/91; 07.02.1991 JP 16327/91
(43) Date of publication of application: 12.08.1992
(73) Proprietor: TERUMO KABUSHIKI KAISHA, Tokyo 151 (JP)
(72) Inventor: Saiki, Masaru, Nakakoma-gun, Yamanashi-ken (JP)
(74) Representative: Hasenrader, Hubert

(56) References cited:
- EP-A- 0 295 075
- WO-A-88/08725
- DE-A- 3 814 023

## Description

### BACKGROUND OF THE INVENTION

The present invention relates to a quantitative dispensing device which can set an arbitrary quantity of medicine filled in a replaceable cartridge and can inject it continuously.

Conventionally, to inject a predetermined quantity of liquid medicine, a syringe is generally used. A syringe of this type is used as, e.g., an insulin syringe for injecting an insulin as a liquid medicine for treatment of diabetes, and a plastic insulin syringe having a needle is widely used.

However, since the plastic insulin syringe is used for home treatment by a patient at home, the waste syringe after use retains the needle and may thus be reused by somebody else, thus posing a social problem.

If a patient need insulin injection several times a day, he or she must always carry the syringe and an ampule of the medicine with him and is thus inconvenient. Furthermore, the fact that he suffers from diabetes can be noticed by others, although he wishes to keep it secret from others.

To replenish the syringe with the medicine from the ampule, the needle must be pierced into the rubber portion of the ampule. As the number of times of use of the needle is increased, the distal end portion of the needle may be broken or the piercing resistance may be increased. This poses a considerable load on the patient physically and mentally.

From the above situation, a pen type portable quantitative dispensing device is proposed which can set an arbitrary quantity of liquid medicine filled in a cartridge and can inject a predetermined quantity of medicine.

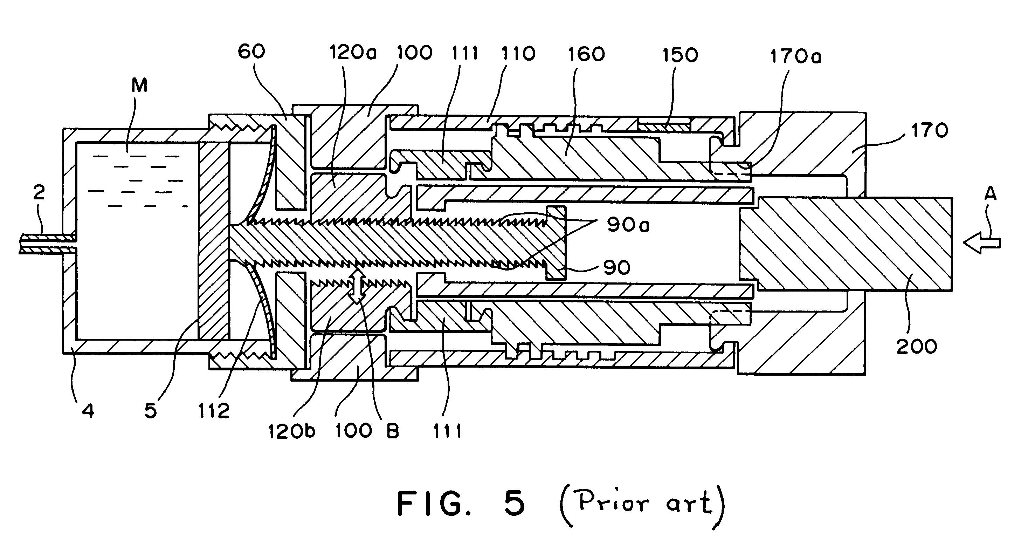

An example of the conventional quantitative dispensing device will be described with reference to the accompanying drawing. Fig. 5 is a central sectional view of a conventional quantitative dispensing device which is disclosed as a "Portable Device for Measuring and Injecting Fluid" by Japanese Patent Laid-Open No. 63-318952 (U.K. Application No. 8,713,810, filed on June 12, 1987).

Referring to Fig. 5, a needle 2 is provided on a cartridge 4 containing a liquid medicine M. A piston 5 is slidable on the inner circumferential surface of the cartridge 4 and hermetically seals the cartridge 4. When a plunger rod 200 is depressed against a plunger 90 in a direction of an arrow A, a liquid medicine M in the cartridge 4 is injected through the needle 2. This is the basic structure of the syringe. In addition to this, the syringe can set an arbitrary quantity of the medicine M filled in the cartridge 4 before each injection and can inject the medicine continuously.

For this purpose, locking ratchets 90a as shown in Fig. 5 are formed on the upper and lower surfaces of the plunger 90 in the longitudinal direction. A locking member 112 is locked with the locking ratchets 90a to enable a movement of the plunger 90 only in a direction to urge the piston 5. The locking member 112 disables a reverse movement of the plunger 90. Therefore, the plunger 90 can be held at a shift position of the piston 5 when the piston 5 is moved along with consumption of the medicine M.

Jaws 120a and 120b are disposed around the plunger 90. The jaws 120 and 120b are moved in a direction of an arrow B in Fig. 5 by a pivot movement of a sleeve 100 and set in locked and unlocked states with and from the locking ratchets of the plunger 90.

Assume that the jaws 120a and 120b are seated on the rear surface of the shoulder 60 to be set at zero position from the state after the previous injection. When a sleeve 170 on the rear end of the syringe is rotated clockwise to move an injection quantity displaying sleeve 160 having a scale mark on its outer circumferential surface to a front position, a latch member 111 of the locking member is engaged with the jaws 120a and 120b.

In this state, "zero" is displayed through a scale window 150. Therefore, the sleeve 170 is rotated counterclockwise until a mark representing a desired injection quantity is displayed through the scale window 150. As a result, the jaws 120a and 120b integrally moved with the sleeve 160 are moved backward by a distance necessary for injection.

Since the jaws 120a and 120b are moved by the distance corresponding to the movement of the plunger 90 necessary for injection, the sleeve 100 is pivoted to lock the jaws 120a and 120b on the plunger 90.

Then, the plunger rod 200 is depressed in the direction of the arrow A to move the piston 5, thereby performing injection.

More specifically, when the previous injection is completed, the sleeve 100 is operated to release the locked state of the jaws 120a and 120b against the ratchet portions of the plunger 90. The sleeve 170 on the rear end is operated to move the jaws 120a and 120b backward, thus setting an injection quantity. Then, the sleeve 100 is operated again to lock the jaws 120a and 120b on the plunger 90. Finally, the plunger rod 200 is depressed. The injection quantity must be set before each injection. After injection is completed, the above operation is repeated. When the medicine M is completely consumed, the cartridge 4 is replaced with a new one.

However, according to the above preposition, the injection quantity must be set before each injection, resulting in a complex operation.

Furthermore, once the sleeve is set to the injection side during the injection quantity settingoperation, an erroneous injection quantity is set. Then, an accident in which an erroneous dose of medicine, e.g., insulin is injected occurs.

### SUMMARY OF THE INVENTION

The present invention has therefore been made in view of the above problem, and has as its object to provide a quantitative dispensing device which can be used continuously and which eliminates the necessity of setting an injection quantity before each medicine injection.

It is another object of the present invention to provide a quantitative dispensing device the operability of which is improved and in which an erroneous injection quantity of medicine will not be set.

It is still another object of the present invention to provide a quantitative dispensing device which is disabled to set an injection quantity when the balance or remains in the cartridge is reduced and a liquid medicine necessary for one injection is not left.

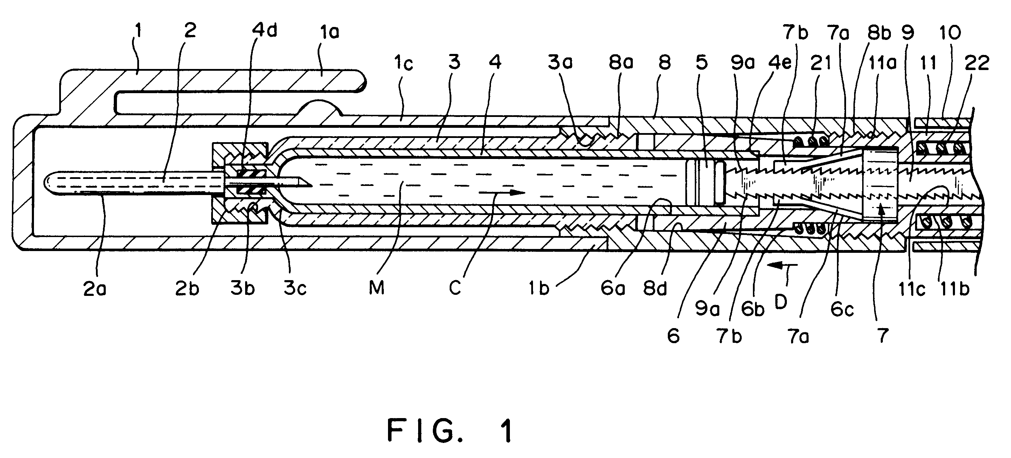

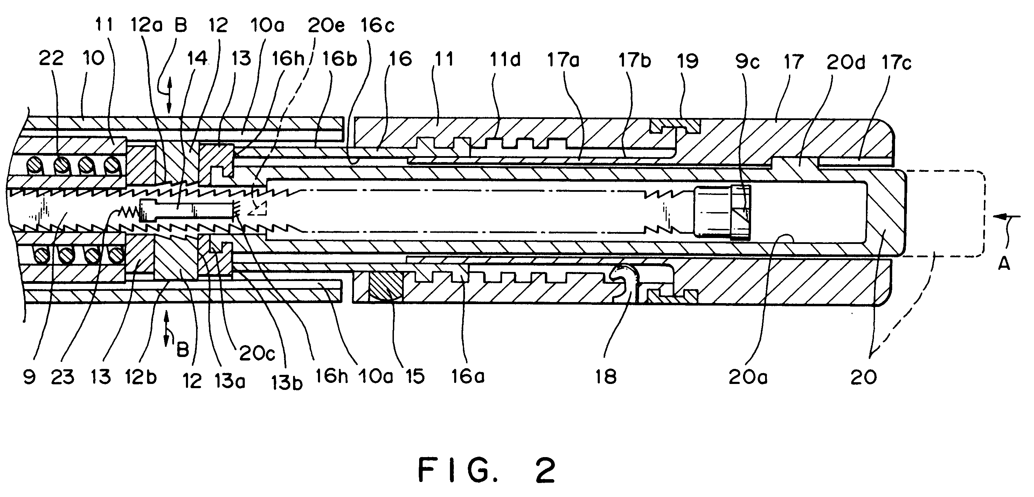

In order to achieve the above objects, according to the present invention, referring to Fig. 1 as the central sectional view showing the front half of a quantitative dispensing device, a cartridge 4 is attached into and detached from a cylindrical main body by a attaching/detaching means so that a piston 5 of the cartridge 4 can be pushed by a plunger 9. The plunger 9 is movable in a direction (direction D) to push the piston 5. Since the plunger 9 is locked with a return preventive member 7 which prohibits its reverse movement, it does not move backward. Referring to Fig. 2 as the central sectional view showing the rear half of the quantitative dispensing device, a pair of jaws 12, which are biased to be set in locked and unlocked states with and from a locking portion 9a of the plunger 9, are held by a jaw holder 13 and movable in the longitudinal direction of the main body. A sleeve 10 is operated to unlock the jaws 12, and a setting member (dial) 17 is operated to set the shift amount of the jaw holder 13 in the longitudinal direction. The shift amount is displayed by a display member 16. A push member 20 for pushing the piston 5 is provided with a jaw holder 13 to be integrally movable with it, and is moved by a biasing means 22 to move the jaw holder 13 toward the display member 16.

It is preferable that a movable piece 14, which is biased to extend between the jaws 12 in order to prohibit movement of the sleeve 10, is provided in the jaw holder 13. Then, when the setting dial 17 is operated to set the shift amount of the jaw holder 13 in the longitudinal direction, the shift amount of the piston 5 upon being pushed by the plunger 9 is prevented from being smaller than a predetermined amount.

It is also preferable to form a locking blade portion 9c at a portion of the plunger 9 opposing the pushing portion of the piston 5, so that it can be locked with a rotation preventive blade portion 20e formed in the push member 20. The push member 20 and the setting dial 17 are interlocked with each other. When the balance in the cartridge 4 is reduced and the shift amount of the piston 5 upon being pushed by the plunger 9 becomes small, the two blade portions are locked with each other to prevent rotation of the setting dial.

Other features and advantages of the present invention will be apparent from the following description taken in conjunction with the accompanying drawings, in which like reference characters designate the same or similar parts throughout the figures thereof.

### BRIEF DESCRIPTION OF THE DRAWINGS

Fig. 1 is a central sectional view showing a front half of a quantitative dispensing device according to an embodiment of the present invention;
Fig. 2 is a central sectional view showing a rear half of the quantitative dispensing device shown in Fig 1;
Fig. 3 is a sectional view of a cartridge receiving member for explaining its operation;
Fig. 4 is a partially cutaway perspective view of the rear half of the quantitative determination injector shown in Fig. 2; and
Fig. 5 is a sectional view of a conventional quantitative dispensing device.

Note that the scope of the present invention is not limited by the embodiment to be described later.

### DETAILED DESCRIPTION OF THE PREFERRED EMBODIMENT

A preferred embodiment of the present invention will be described in detail with reference to the accompanying drawings. Fig. 1 is a central sectional view of the front half of a quantitative dispensing device according to an embodiment of the present invention.

Referring to Fig. 1, a needle 2 is replaceably provided on a transparent cartridge 4 containing a liquid medicine M. The cartridge 4 can be freely set in a transparent cartridge holder 3 as shown in Fig. 1. The needle 2 can be fixed, as shown in Fig. 1, by rotating a thread member 2b threaded with a mouth thread portion 3b of the cartridge holder 3. A needle cap 2a is applied on the needle 2 to protect the distal end portion of the needle 2 and to prevent it from causing a danger when it is disposed after use.

A rubber plug 4d, which can be pierced by the needle 2 so that the cartridge 4 can be handled as it contains the liquid medicine M, is provided in the left end of the cartridge 4. A piston 5, which is freely slidable on the inner circumferential surface of the cartridge 4 in the longitudinal direction, thus keeping the hermetic state to push the medicine M to the outside, is inserted in the right end of the cartridge 4.

The cartridge holder 3 forms an inner circumferential surface substantially corresponding to the outer shape of the cartridge 4 with a clearance gap, as shown in Fig. 1, and has a length shorter than that of the cartridge 4.

The mouth thread portion 3b threadably engaged with the thread member 2b to retain the needle 2 is formed on the outer circumferential surface of the left end of the cartridge holder 3. A male thread portion 3a, threadable with a first female portion 8a of a barrel 8 to mount the cartridge holder 3 on the barrel 8, is formed on the outer circumferential surface of the right end of the cartridge holder 3.

A cap 1 which can be fitted on a fitting portion 1b is provided on the barrel 8. When the injector is not in use, the cap 1 is fitted as shown in Fig. 1 to protect the needle 2, thus ensuring portability. When the injector is kept in a pocket with its clip portion 1 being hooked on the pocket, the quantitative dispensing device does not appear as such from the outside.

When the cartridge 4 containing the liquid medicine M is emptied, the cap 1 is removed, the cartridge holder 3 is held, and the thread member 2b is loosened to remove the needle 2 capped with the needle cap or sheath 2a by pulling it out from the rubber plug 4d of the cartridge 4. The sheath 2a is made of rubber.

Then, the cartridge holder 3 is pivoted while it is held, to unlock it from the barrel 8, and is removed from the barrel 8. A new cartridge 4 filled with the medicine M is set in the cartridge holder 3.

The male thread portion 3a of the cartridge holder 3 is threadably engaged with the first female thread portion 8a of the barrel 8, a plunger 9 urging the piston 5 is inserted in the cartridge 4, and the cartridge holder 3 is pivoted.

As a result, an inner circumferential R portion 3c of the cartridge holder 3 is urged against the cartridge 4 to move it in a direction of an arrow C in Fig. 1, and a right end portion 4e of the cartridge 4 acts on a cartridge receiving member 6. Replacement of the cartridges 4 is completed by the above operation. An injection preparation for the piston 5 is subsequently performed.

The cartridge receiving member 6 is provided in an inner hole portion 8d of the barrel 8 to be movable in the longitudinal direction. The cartridge receiving member 6 is biased in a direction of an arrow D in Fig. 1 by compression of a first urging member or push spring 21 provided between a step portion 6b of the cartridge receiving member 6 and a male thread portion 11a of an inner barrel 11.

The cartridge receiving member 6 receives the plunger 9 and forms a pair of taper portions 6c on its inner circumferential surface, as shown in Fig. 1. The outer side of a locking portion 7b of a return preventive member 7 of the plunger 9 contacts the taper portions 6c. When the cartridge 4 is inserted as shown in Fig. 1, the locking portion 7b of the return preventive member 7 keeps the plunger 9 at a predetermined position.

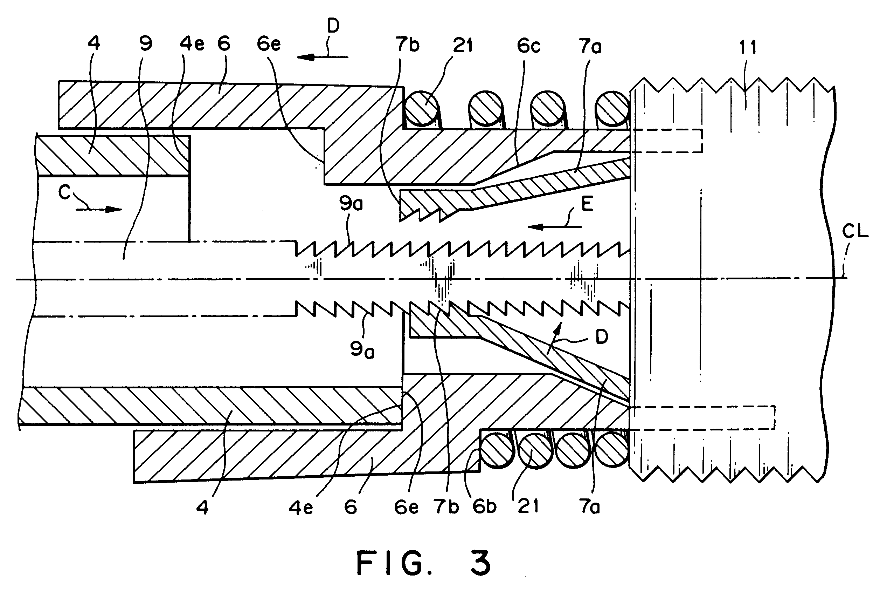

Fig. 3 is a view showing the operation of the return preventive member 7 for the plunger 9. The portion above a central line CL indicates a state before setting, and a portion below the central line CL indicates a state after setting which is similar to that of Fig. 1.

The operation of the return preventive member 7 will be described. The plunger 9 urging the piston 5 as described above is inserted in the cartridge 4. When the cartridge holder 3 is pivoted, the inner circumferential R portion 3c of the cartridge holder 3 starts to move within the cartridge 4 in a direction indicated by an arrow C in Fig. 3. At this time, the locking portion 7b of the return preventive member 7 is not locked with a locking portion 9a of the plunger 9 because of its own elastic force.

When the cartridge holder 3 is further pivoted to move in the direction of the arrow C, the right end portion 4e of the cartridge 4 is brought into contact with a bottom portion 6e of the cartridge receiving member 6. When the cartridge holder 3 is still further pivoted to move in the direction of the arrow C, the taper portions 6c move the locking portion 7b of the return preventive member 7 in a direction of an arrow D in Fig. 3 to lock the locking portion 9a of the plunger 9 with the locking portion 7b of the return preventive member 7.

The locked state between the locking portion 9a and the locking portion 7b of the return preventive member 7 constitutes a bellows joint. The plunger 9 constitutes a uni-directional return preventive mechanism which can move toward the left as indicated by an arrow E in Fig. 3 (in a direction to urge the piston 5) but cannot move backward.

The return preventive member 7 for the plunger 9 is not limited to the above arrangement but can be of any type as far as it is a return preventive mechanism using a gear mechanism or a ring.

When the cartridge 4 is set in the manner as described above, the plunger 9 is set movable only in a direction to urge the piston 5 and immovable in the reverse direction. Thus, the plunger 9 can be kept at a shift position of the piston 5 as the medicine M is consumed.

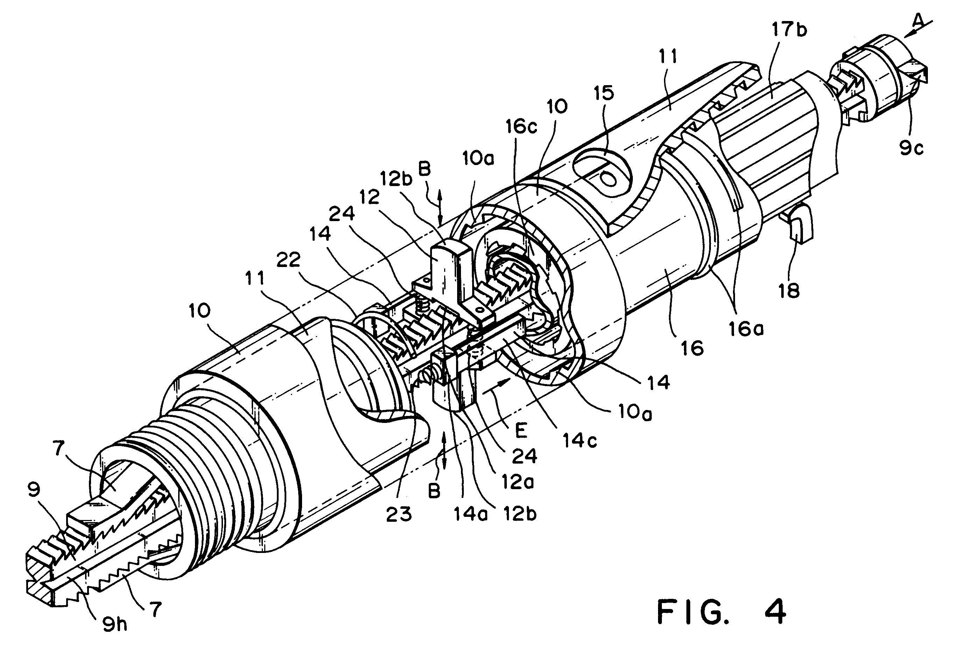

A mechanism for allowing an injection quantity setting operation and an injection operation of the plunger 9 will be described with reference to the sectional and partially cutaway perspective views of Figs. 2 and 4.

A sleeve 10 for performing switching between injection and injection quantity setting operation is provided for the plunger 9. A pair of projections 10a are formed on the inner circumferential surface of the sleeve 10, and the sleeve 10 is pivotal about the inner barrel 11. Jaws 12 are provided in the inner barrel 11. Upon operation of the projections 10a, the jaws 12 are moved in a direction of an arrow B in Fig. 2 or 4 to be locked with and unlocked from the locking portion 9a of the plunger 9.

A mechanism for locking and unlocking the jaws 12 with and from the plunger 9 by pivotally operating the sleeve 10 will be described. Referring to Fig. 4, the jaws 12 oppose each other and are held by a jaw holder 13 (shown in Fig. 2). The compression force of fourth push springs 24 acts on the jaws 12 to bias them toward the outside.

The jaws 12 are biased by the fourth urging member or push springs 24 to be disengaged from the plunger 9. As the sleeve 10 is pivoted, its projections 10a are brought into contact with an outer circumferential surfaces 12b of the jaws 12 to lock the jaws 12 with the plunger 9.

Balance safety pieces 14 are provided between the jaws 12 and biased by the compression force of third urging member or push springs 23 in a direction indicated by an arrow E in Fig. 4. Proximal portions 14a of the balance safety pieces 14 extend between the jaws 12 in order to prevent the jaws 12 from being engaged with the plunger 9. A piece portion 14c is integrally formed on each balance safety piece 14. When the end portions of the piece portions 14c are brought into contact with and moved by a side surface portion 16h of an injection quantity display member 16 to be described later, the balance safety pieces 14 are moved in a direction opposite to that indicated by the arrow E, thereby locking the jaws 12 with the plunger 9.

Referring again to Fig. 2, a distance between the side surface portion 16h of the injection quantity display member 16 and a side surface portion 13b of the jaw holder 13 defines the shift amount of the plunger 9 and thus corresponds to the injection quantity of the medicine M. Therefore, when the sleeve 10 is set to the quantity setting operation side to disengage the jaws 12, the jaw holder 13 is automatically moved to a position to contact the side surface portion 16h of the injection quantity display member 16 by the operation of a second urging member or push spring 22 stored in the inner barrel 11.

A push member 20 depressed in a direction of an arrow A in Fig. 2 is integrally formed with the jaw holder 13. Therefore, when the jaw holder 13 is moved, the push member 20 is also moved in an interlocked manner by a distance corresponding to a preset injection quantity. In this state, when the sleeve 10 is set to the injection side, the plunger 9 and the jaws 12 are locked with each other. When the push member 20 is depressed in the direction of the arrow A, the jaw holder 13 and the jaws 12 are moved in an interlocked manner, and the plunger 9 is moved in a direction to urge against the piston 5 of the cartridge 4.

The shift distance of the jaw holder 13 is regulated by the end portion of the inner barrel 11 so as not to move over it. The plunger 9 is moved by a distance corresponding to a preset injection quantity by the above operation.

The injection quantity is determined by rotating an injection quantity setting dial 17 in Fig. 2. The injection quantity setting dial 17 has an outer circumferential spline 17b. A click unit 18 contacts the recesses and projections of the spline 17b to control the rotational amount.

A trapezoidal thread 11d is formed on the inner circumferential surface of the inner barrel 11, and a trapezoidal thread 16a of the injection quantity display member 16 is threadably engaged with the trapezoidal thread 11d. When the injection quantity display member 16 is pivoted, it is moved in the longitudinal direction. For this purpose, the injection quantity display member 16 is connected to the injection quantity setting dial 17 through a spline structure. When the injection quantity setting dial 17 connected to the inner barrel 11 through a connecting member 19 is rotated, the injection quantity display member 16 is rotated and moved in the axial direction by the operation of the thread.

At this time, as the figures marked on the outercircumferential surface of the injection quantity display member 16 are moved by the rotating and axial movements of the display member 16, the value externally seen through an injection quantity display window 15 of the inner barrel 11 changes. The injection quantity display window 15 is constituted by a lens and the size of the figure which can be seen by the naked eye is enlarged to be larger than it actually is.

The quantity of medicine M that can be filled in the cartridge 4 is limited. When the balance of the medicine M in the cartridge 4 is smaller than the preset injection quantity, the balance safety pieces 14 function in the manner as described above, so that the sleeve 10 which performs switching between setting and injection cannot be operated from the setting operation side to the injection side.

In addition to the balance safety pieces 14, the injection quantity setting dial 17 has a mechanism which prevents rotation of the injection quantity setting dial 17 when the balance of the medicine M in the cartridge 4 is smaller than the preset injection quantity.

This mechanism will be described. Referring to Fig. 2, as the medicine M in the cartridge 4 is consumed, the plunger 9 is moved to the left until a blade portion 9c formed on its end portion is locked with a blade portion 20e formed on the interior of the push member 20. A recess 17c is formed in the injection quantity setting dial 17. A projection 20d on the outer circumferential surface of the push member 20 is integrally moved with the recess 17c in the longitudinal and radial directions. When the injection quantity setting dial 17 is rotated, the push member 20 is rotated simultaneously. Thus, when the blade portion 9c is moved to the position to lock with the blade portion 20e formed on the inner surface of the push member 20, the push member 20 is disabled to rotate. Therefore, the injection quantity setting dial 17 cannot be rotated.

The operation of the injector having the above arrangement will be described. The cap 1 is removed and the sleeve 10 is held and pivoted to the setting operation side to disengage the pair of jaws 12 from the plunger 9. The cartridge 4 is inserted in the cartridge holder 3 and mounted on the barrel 8, and the needle 2 is mounted on the cartridge 4.

In this state, since air is contained in the interior of each of the cartridge 4 and the needle 2, the injection quantity setting dial 17 is rotated by an appropriate amount (one click being set as two units). Then, the push member 20 is moved backward simultaneously in a direction opposite to that of the arrow A.

The sleeve 10 is pivoted to the injection side while the distal end of the needle 2 is kept pointing upward. The push member 20 is slowly depressed in the direction of the arrow A until the medicine M flows from the needle point, thus removing air in the cartridge 4 and the needle 2. Thereafter, the sleeve 10 is set to the setting operation side, the injection quantity setting dial 17 is rotated to determine the injection quantity with reference to the figure marked on the outer circumferential surface of the injection quantity display member 16, and the sleeve 10 is set to the injection side.

If the injection quantity is already set when air is removed and the injection quantity setting dial 17 need not be operated, the sleeve 10 is set to the setting operation side. Then, when the push member 20 projects, the sleeve 10 only need be switched to the injection side at once. The needle 2 of the injector is pierced through the patient's skin in this state. The medicine in the preset injection quantity is injected by pushing the push member 20 completely.

When injection is to be performed again, the sleeve 10 only need be set to the setting operation side and then subsequently switched to the injection side. When an injection quantity is to be changed, while the sleeve 10 is set in the setting operation side, the injection quantity setting dial 17 is rotated to change an injection quantity (at this time, it is apparent from the structure described above that the injection quantity setting dial 17 need not be returned to a predetermined position and that an injection quantity can be freely increased or decreased.)

When the needle 2 or the cartridge 4 is replaced, the air removing operation is performed so that air is not injected together with the medicine M. However, it is preferable to preset the injection quantity in advance, to perform the air removing operation, and thereafter to operate only the sleeve 10, because the operability is improved in this case.

When the balance of the medicine M in the cartridge 4 is smaller than the preset quantity, the balance safety pieces 14 described above prevent the jaws 12 from being locked with the plunger 9, and thus the sleeve 10 cannot be operated to the injection side. Erroneous injection can thus be prevented. In this case, the injection quantity setting dial 17 is returned to the "zero" point, and the injection quantity is set. A position where the injection quantity setting dial 17 becomes immovable indicates the balance of the medicine M in the cartridge 4. If this balance is insufficient, the cartridges 4 are replaced and the medicine in a quantity corresponding to a shortage is injected.

The dispensing device of the present invention can comprise an audible alarm member such as a buzzer or an visible alarm member such as a liquid crystal display, when the balance of the medicine is smaller than the predetermined quantity.

Further, the injection quantity can be displayed by a liquid crystal display. Further, the dispensing device of the present invention can comprise a memory member to store the last time and day when the medicine was injected the last and an alarm member to inform the time and day when the medicine must be injected. The alarm member is preferably used an audible alarm member such as a buzzer or an visible alarm member such as a liquid crystal display.

The dispensing device of the present invention can comprise a clock member which displays the present time

As has been described above, when compared to a conventional syringe to which insulin is directly transferred, an error in injection quantity caused by structural or human factor is eliminated, and the medicine in a correct quantity can be injected.

Because of the operation of the first push spring 21 in the main body, once an injection quantity is set, selection between the setting operation and the injection can be performed only by operating the sleeve 10. When the injection quantity is to be changed, it can be freely increased or decreased from the preset quantity. Therefore, the operability is remarkably improved when compared even with the conventional cartridge type injector.

Once the needle 2 is pierced in the patient's skin, it is preferable to complete injection only by one depression operation. For this purpose, the injection quantity display member 16 is provided, and an injection quantity of the medicine to be injected can be changed by a single operation of the setting dial 17. Therefore, an arbitrary quantity of medicine can be injected by one depression.

The internal connection structure of the main body and the spline coupling structure are combined to eliminate the danger in injection that a displayed injection quantity is different from the quantity of medicine actually injected from the needle.

A safety structure is employed in which the balance safety pieces are provided. Therefore, when the balance of the medicine in the cartridge 4 is smaller than the preset injection quantity, injection cannot be performed.

According to the present invention, there is provided a quantitative dispensing device in which the need for setting the injection quantity before each injection is eliminated, as described above, thus improve the operability, and with which an erroneous injection quantity of medicine is not injected.

There is also provided a quantitative dispensing device which can prevent an injection quantity setting operation when the balance in the cartridge is reduced.

As many apparently widely different embodiments of the present invention can be made without departing from the scope thereof, it is to be understood that the invention is not limited to the specific embodiments thereof except as defined in the appended claims.

## Claims

1. A quantitative dispensing device for injecting a liquid medicine in a predetermined quantity by using a cartridge (4), said device comprising the cartridge containing the liquid medicine, a piston (5) slidably provided in said cartridge, and a needle (2) for causing an interior and exterior of said cartridge to communicate with each other, said device also comprising
a cylindrical main body, (8, 10, 11)
attaching/detaching means (6) for freely attaching and detaching said cartridge at one end of said main body,
return preventive means (7), held to be movable in a longitudinal direction of said main body, and acting on a locking portion of a plunger to move and hold said plunger so as to be movable in a direction to urge said piston and immovable in a reverse direction,
locking means (12) set in a locked state and an unlocked state with respect to said locking portion of said plunger,
a setting member (17), pivotally provided at the other end of said main body in order to set a shift amount of said plunger in the longitudinal direction when said locking means is in the unlocked state, and
a display member (16), moved in the longitudinal direction by pivot adjustment of said setting member, for displaying the shift amount, characterized in that said device further comprises an
urging member (21) provided to be substantially coaxial with said setting member and movable in the longitudinal direction; and
a second urging member (22) for integrally moving the push member (20) and said locking means and biasing said locking means toward said setting member (17) in said main body.

2. An injector according to claim 1, wherein said locking means (12) further include a balance safety piece (14) having a biasing force to prohibit movement of said setting member (17) and extending between said locking means (12).

3. An injector according to claim 1 or 2, wherein a locking blade portion (9c) is provided at a rear end of said plunger (9), a locking blade portion (20e) to be locked with said locking blade portion (9c) to prevent rotation of said push member (20) is formed in said push member (20), and said push member and said setting member (17) are interlocked with each other.

4. An injector according to anyone of claims 1 to 3, wherein said locking means (12) comprise:
a pair of jaw members (12) having a biasing force acting toward an outer circumference of said main body so that said jaw members are set in the unlocked state in which said locking means (12) are not locked with said locking portion (9a) of said plunger,
a jaw member holder (13) for holding said jaw members and movable in said main body in the longitudinal direction; and
a sleeve member (10), forming an inner circumferential cam portion (10a) which moves said jaw members against the biasing force to the locked state in order to select and set either the locked or unlocked state, and pivotally provided about said main body.

5. An injector according to anyone of claims 1 to 4, wherein said attaching/detaching means (6) is moved upon insertion of said cartridge and is interlocked with said return preventive means to be locked with said locking portion of said plunger.

6. An injector according to anyone of claims 1 to 5, further comprising a cartridge holder (3) threadably engaged with said main body in order to allow insertion of said cartridge of said attaching/detaching means (6).

7. An injector according to anyone of claims 1 to 6, wherein the liquid medicine is an insulin.

8. An injector according to anyone of claims 1 to 7, wherein a cap (1) for protecting said needle is detachably mounted on said main body.

9. An injector according to anyone of claims 1 to 8, wherein a click member for moving and holding a display member (16) in a stepwise manner is provided, said display member being moved in the longitudinal direction by pivot adjustment of said setting member (17) in order to display the shift amount.

10. An injector according to anyone of claims 1 to 9, wherein a lens (15) for enlarging and displaying a display portion of said display member for displaying the shift amount is provided in said main body.

## Patentansprüche

1. Dosiervorrichtung zum Injizieren von flüssiger Arznei in einer vorbestimmten Menge durch die Verwendung einer Patrone (4), wobei die Vorrichtung aufweist: die die flüssige Arznei enthaltende Patrone, einen in der Patrone gleitfähig vorgesehenen Kolben (5) und eine Nadel (2), um das Innere und Äußere der Patrone miteinander zu verbinden, wobei die Vorrichtung ebenfalls aufweist:
einen zylindrischen Hauptkörper (8, 10, 11),
eine Befestigungs/Löseeinrichtung (6), um die Patrone an einem Ende des Hauptkörpers unbehindert zu befestigen und zu lösen,
eine Rückbewegungs-Verhinderungseinrichtung (7), die in Längsrichtung des Hauptkörpers beweglich gehalten ist und auf einen Arretierabschnitt eines Plungerkolbens wirkt, um den Plungerkolben zu bewegen und zu halten, so daß dieser in eine Richtung zum Spannen des Kolbens beweglich ist und in umgekehrte Richtung nicht beweglich ist,
Arretiereinrichtungen, die in bezug auf den Arretierabschnitt des Plungerkolbens in einen arretierten Zustand und einen nicht arretierten Zustand eingestellt sind,
ein Einstellelement (17), das am anderen Ende des Hauptkörpers drehbar vorgesehen ist, um, wenn sich die Arretiereinrichtungen im entarretierten Zustand befinden, den Verschiebungsbetrag des Plungerkolbens in Längsrichtung einzustellen, und
ein Anzeigeelement (16), das durch Drehregulierung des Einstellelements in Längsrichtung bewegt wird, zum Anzeigen des Verschiebungsbetrages,
**dadurch gekennzeichnet, daß**
die Vorrichtung ferner aufweist:
ein Spannelement (21), das mit dem Einstellelement im wesentlichen koaxial verläuft und in Längsrichtung beweglich vorgesehen ist, und
ein zweites Spannelement (22), um das Drückelement (20) und die Arretiereinrichtungen einstückig zu bewegen und im Hauptkörper die Arretiereinrichtungen zum Einstellelement (17) hin vorzuspannen.

2. Injektor nach Anspruch 1, wobei die Arretiereinrichtungen (12) ferner ein Restbetrag-Sicherungsstück (14) aufweisen, das zum Verhindern der Bewegung des Einstellelements (17) eine Vorspannkraft aufweist und sich zwischen den Arretiereinrichtungen (12) erstreckt.

3. Injektor nach Anspruch 1 oder 2, wobei ein flügelartiger Arretierabschnitt (9c) am hinteren Ende des Plungerkolbens (9) vorgesehen ist, ein flügelartiger Arretierabschnitt (20e), der zum Verhindern der Drehung des Drückelements (20) mit dem flügelartigen Arretierabschnitt (9c) zu arretieren ist, im Drückelement (20) ausgebildet ist und das Drückelement und das Einstellelement (17) miteinander arretiert sind.

4. Injektor nach einem der Ansprüche 1 bis 3, wobei die Arretiereinrichtungen (12) aufweisen:
ein Paar von Backenelementen (12) mit einer Vorspannkraft, die zum Außenumfang des Hauptkörpers wirkt, so daß die Backenelementen in den nicht arretierten Zustand eingestellt sind, in dem die Arretiereinrichtungen (12) mit dem Arretierabschnitt (9a) des Plungerkolbens nicht arretiert sind,
eine Backenelement-Halteeinrichtung (13) zum Halten der Backenelemente, die im Hauptkörper in Längsrichtung beweglich ist, und
ein Buchsenelement (10), das einen Innenumfangs-Nockenabschnitt (10a) bildet, der das Backenelement entgegen der Vorspannkraft in den arretierten Zustand bewegt,um den arretierten oder den nicht arretierten Zustand auszuwählen und einzustellen, und um den Hauptkörper herum drehbar vorgesehen ist.

5. Injektor nach einem der Ansprüche 1 bis 4, wobei die Befestigungs/Löseeinrichtung (6) bei Einführen der Patrone bewegt wird und mit der Rückbewegungs-Verhinderungseinrichtung arretiert wird, um mit dem Arretierabschnitt des Plungerkolbens arretiert zu sein.

6. Injektor nach einem der Ansprüche 1 bis 5, der ferner eine Patronenhalteeinrichtung (3) aufweist, die mit dem Hauptkörper in Schraub-Eingriff steht, um das Einführen der Patrone in die Befestigungs/Löseeinrichtung (6) zu gestatten.

7. Injektor nach einem der Ansprüche 1 bis 6, wobei die flüssige Arznei Insulin ist.

8. Injektor nach einem der Ansprüche 1 bis 7, wobei eine Kappe (1) zum Schutz der Nadel am Hauptkörper lösbar montiert ist.

9. Injektor nach einem der Ansprüche 1 bis 8, wobei ein Einrastelement zum schrittweisen Bewegen und Halten eines Anzeigeelements (16) vorgesehen ist, wobei das Anzeigeelement durch Drehregulierung des Einstellelements (17) in Längsrichtung bewegt wird, um den Verschiebebetrag anzuzeigen.

10. Injektor nach einem der Ansprüche 1 bis 9, wobei eine Linse (15) zum Vergrößern und Anzeigen eines Anzeigeabschnitts des Anzeigeelements zum Anzeigen des Verschiebebetrages im Hauptkörper vorgesehen ist.

## Revendications

1. Dispositif de distribution quantitative pour injecter un médicament liquide en une qualité prédéterminée au moyen d'une cartouche (4), ledit dispositif comprenant la cartouche contenant le médicament liquide, un piston (5) prévu coulissant dans ladite cartouche et une aiguille (2) pour amener l'intérieur et l'extérieur de ladite cartouche à communiquer entre eux, ledit dispositif comprenant en outre :
un corps principal cylindrique (8, 10, 11),
un moyen de fixation/séparation (6) pour fixer et séparer librement ladite cartouche à une extrémité dudit col principal,
un moyen anti-retour (7) maintenu pour être déplaçable dans une direction longitudinale dudit corps principal, et agissant sur une partie de blocage d'un plongeur pour déplacer et maintenir ledit plongeur pour qu'il soit déplaçable dans une direction pour solliciter ledit piston et pour qu'il soit non déplaçable dans une direction opposée,
des moyens de blocage (12) réglés dans un état bloqué et dans un état non bloqué par rapport à ladite partie de blocage dudit plongeur,
un élément de réglage (17) prévu de manière pivotante à l'autre extrémité dudit corps principal pour régler une ampleur de décalage dudit plongeur dans la direction longitudinale lorsque lesdits moyens de blocage sont dans l'état non bloqué, et
un élément d'affichage (16) déplacé dans la direction longitudinale par un réglage à pivotement dudit élément de réglage, pour afficher l'ampleur de décalage, caractérisé en ce que ledit dispositif comprend en outre
un élément de sollicitation (21) prévu pour être sensiblement coaxial avec ledit élément de réglage et pour être déplaçable dans la direction longitudinale ; et
un second élément de sollicitation (22) pour déplacer de manière solidaire l'élément de poussée (20) et lesdits moyens de blocage et pour solliciter lesdits moyens de blocage en direction dudit élément de réglage (17) dans ledit corps principal.

2. Injecteur selon la revendication 1, dans lequel lesdits moyens de blocage (12) comprennent en outre une pièce de sécurité de reste (14) ayant une force de sollicitation pour empêcher le mouvement dudit élément de réglage (17) et qui s'étend entre lesdits moyens de blocage (12).

3. Injecteur selon la revendication 1 ou 2, dans lequel une partie formant lame de blocage (9c) est prévue à une extrémité arrière dudit plongeur (9), une partie formant lame de blocage (20e) destinée à être bloquée avec ladite partie formant lame de blocage (9c) pour empêcher la rotation dudit élément de poussée (20) est formée dans ledit élément de poussée (20), et ledit élément de poussée et ledit élément de réglage (17) sont mutuellement bloqués.

4. Injecteur selon l'une quelconque des revendications 1 à 3, dans lequel lesdits moyens de blocage (12) comprennent :
une paire d'éléments formant mâchoires (12) ayant une force de sollicitation agissant en direction d'une circonférence externe dudit corps principal de sorte que lesdits éléments formant mâchoires sont réglés dans l'état non bloqué dans lequel lesdits moyens de blocage (12) ne sont pas bloqués avec ladite partie de blocage (9a) dudit plongeur,
un support d'éléments formant mâchoires (13) pour maintenir lesdits éléments formant mâchoires et déplaçable dans ledit corps principal dans la direction longitudinale ; et
un élément formant manchon (10) formant une partie formant came circonférentielle interne (10a) qui déplace lesdits éléments formant mâchoires contre la force de sollicitation dans l'état bloqué afin de choisir et régler l'état bloqué ou non bloqué, et qui est monté à pivotement autour dudit corps principal.

5. Injecteur selon l'une quelconque des revendications 1 à 4, dans lequel ledit moyen de fixation/séparation (6) est déplacé par insertion de ladite cartouche et est bloqué mutuellement avec ledit moyen anti-retour pour être bloqué avec ladite partie de blocage dudit plongeur.

6. Injecteur selon l'une quelconque des revendications 1 à 5, comprenant en outre un support de cartouche (3) coopérant par vissage avec ledit corps principal pour permettre l'insertion de ladite cartouche dudit moyen de fixation/séparation (6).

7. Injecteur selon l'une quelconque des revendications 1 à 6, dans lequel le médicament liquide est une insuline.

8. Injecteur selon l'une quelconque des revendications 1 à 7, dans lequel un capuchon (1) pour protéger ladite aiguille est monté de manière amovible sur ledit corps principal.

9. Injecteur selon l'une quelconque des revendications 1 à 8, dans lequel il est prévu un élément d'enclenchement pour déplacer et maintenir par pas un élément d'affichage (16), ledit élément d'affichage étant déplacé dans la direction longitudinale par réglage à pivotement dudit élément de réglage (17) afin d'afficher l'ampleur de décalage.

10. Injecteur selon l'une quelconque des revendications 1 à 9, dans lequel une lentille (15) pour agrandir et afficher une partie d'affichage dudit élément d'affichage pour afficher l'ampleur de décalage est prévue dans ledit corps principal.
